# EUROPÄISCHE PATENTSCHRIFT

(11) **EP 2 477 742 B1**
(45) Veröffentlichungstag und Bekanntmachung des Hinweises auf die Patenterteilung: **01.05.2013**
(21) Anmeldenummer: 10752836.6
(22) Anmeldetag: 15.09.2010
(51) Int. Cl.: B01L 3/00

(54) **PROBENEINGABEVORRICHTUNG ZUR EINGABE VON FLÜSSIGEN PROBEN (CLOT CATCHER)**
SAMPLE INPUT DEVICE FOR INPUTTING LIQUID SAMPLES (CLOT CATCHER)
DISPOSITIF DE TRANSFERT D'ÉCHANTILLON POUR TRANSFÉRER DES ÉCHANTILLONS LIQUIDES (DISPOSITIF D'INTERCEPTION DE CAILLOT)

(30) Priorität: 17.09.2009 AT 14742009
(43) Veröffentlichungstag der Anmeldung: 25.07.2012
(73) Patentinhaber: Hoffmann-La Roche AG, 4070 Basel (CH)
(72) Erfinder: EGGER, Felix, 8010 Graz (AT); GULO, Stefan, 8054 Riederhof (AT)
(74) Vertreter: Margotti, Herwig Franz
(86) Internationale Anmeldenummer: PCT/EP2010/063578
(87) Internationale Veröffentlichungsnummer: WO 2011/033000

(56) Entgegenhaltungen:
- EP-A2- 0 573 884
- DE-A1- 19 949 561
- DE-U1-202006 010 970
- ANONYMOUS: "Mini-Spike Medikamentenzubereitung war nie einfacher...", B. Braun Melsungen AG Produkt Information, 14. Januar 2009 (2009-01-14), XP002625059, Gefunden im Internet: URL:http://www.bbraun.de/service-layer-cor e/res/public/document/BPR00000000000000010 0002212300000/49DDA58998060062E1008000D400 106F49DDA58B98060062E1008000D400106F?vc=CO RPORATE_WEBSITE_DE&s=e155d3c79984cf1c312ce 1da1b7695e6 [gefunden am 2011-02-25]

## Beschreibung

Diese Erfindung betrifft Probeneingabevorrichtungen zur Eingabe von flüssigen Proben, insbesondere Blutproben, aus Probengefäßen, insbesondere Spritzen, in Analysatoren zur Untersuchung der Zusammensetzung dieser Proben, gemäß dem Oberbegriff des Anspruchs 1. Aus den Dokumenen "Mini-Spike", 14.1.2009, B. Braun Melsungen AG, XP002625059 und EP 0 573 884 A2 sind Zuspritz- und Entnahmespikes bekannt, die für die Zuführung von flüssigen Proben aus Probegefäßen in ein Analysegerät geeignet sind. Die Erfindung betrifft insbesondere Probeneingabevorrichtungen zur Eingabe von medizinischen Proben, vorzugsweise Vollblut, Serum und Plasma, aus unterschiedlichen Probegefäßen in Analysatoren für die Blutanalyse.

Beispielhafte derartige Analysatoren sind Blutgas-Analysatoren, welche beispielsweise als portable Analysatoren zur Bestimmung von POC (Point Of Care) Parametern, beispielsweise der Blutgase (O₂, CO₂, pH), der Elektrolyte (z.B. K⁺, Na⁺, Ca⁺⁺, Cl⁻) der Metabolite (z.B. Glukose und Laktat), des Hämatokrits, der Hämoglobinparameter (z.B. tHb, SO₂, etc.) und Bilirubin entwickelt wurden und welche vor allem zur dezentralen und raschen Bestimmung der oben genannten Parameter in Vollblutproben eingesetzt werden. Derartige Analysatoren sind beispielsweise die cobas® b 123 und cobas® b 221 Systeme von Roche Diagnostics. Analoge Anwendungen in der Veterinärmedizin und die Verwendung von Serum-, Plasma-, Harn- und Dialysat-Proben sind ebenfalls bekannt.

Zum Teil sind diese Analysatoren lediglich auf eine Art von Probegefäß spezialisiert, sodass deren geräteseitige Eingabevorrichtungen die Eingabe der Probe beispielsweise nur mit Hilfe einer Spritze oder einer Kapillare erlaubt.

Gängige Probengefäße, welche zum Transport von Proben vom Entnahmeort der Probe zum Analysator und dessen geräteseitiger Probeneingabe genutzt werden, sind insbesondere für Blutproben:
- Spritzen: Spritzen, insbesondere speziell adaptierte Blutgasanalysespritzen, sind sowohl aus Kunststoff als auch aus Glas am Markt erhältlich und unterscheiden sich durch das mögliche Füllvolumen (ca. 1 ml bis 20 ml) und das verwendete Antikoagulans. Der Konusbereich der Spritzen, an den zur Blutabnahme die Nadel angesteckt wird, ist durch die Luer-Norm standardisiert (DIN-EN20594-1; EN1707; EN20594-1). Weiters ist der minimale Innendurchmesser dieses Luer-Konus in der Norm für sterile Einmal-Spritzen (EN ISO 7886-1) definiert. Aufgrund des großen Füllvolumens können oft mehrere Messungen aus derselben Spritze durchgeführt werden.
- Kapillaren: Kapillaren, insbesondere zur Blutgasanalyse, sind sowohl aus Kunststoff als auch aus Glas am Markt erhältlich und unterscheiden sich wiederum durch das mögliche Füllvolumen und das verwendete Antikoagulans. Das Füllvolumen ist jedoch deutlich geringer als bei Spritzen (ca. 50 µL bis 250 µL) und ermöglicht daher meist nur eine Analyse pro Probengefäß. Weiters ist der nutzbare Außendurchmesser der Kapillaren abhängig vom Füllvolumen und dem verwendeten Analysator.

Die geräteseitige Probeneingabe bei herkömmlichen Analysegeräten kann in zwei Hauptgruppen unterteilt werden:
- Einfüllmund: Der Einfüllmund besteht zumeist aus einem weichen Kunststoff, an den je nach Analysator Kapillaren und/oder Spritzen angesteckt werden können. Je nach Analysator und Betriebsweise sind dabei unterschiedliche Eingabemodi möglich:
   - Einsaugen: ist möglich aus Kapillaren (z.B. am cobas® b 123 von Roche Diagnostics). Die Kapillare wird an den Einfüllmund angesteckt. Anschließend wird die Probe durch den Analysator automatisch eingesaugt.
   - Einspritzen: ist möglich mit Spritzen (z.B. am cobas® b 221 von Roche Diagnostics): Die Spritze wird an den Einfüllmund angedrückt. Anschließend wird die Probe durch den Bediener aktiv eingespritzt.
- Einsaugnadel: Manche Blutgasanalysatoren, wie der cobas® b 221 und der cobas® b 123, sind alleinig oder in Kombination mit einem Einfüllmund mit einer ausfahrbaren Nadel (Stahlrohr) ausgestattet. Dieses Rohr wird in die Öffnung des Probengefäßes, beispielsweise einer Spritze, eingeführt. Abhängig vom Analysator erfolgt das Einführen des Rohres entweder manuell durch den Bediener oder automatisch durch den Analysator. Anschließend saugt der Analysator die Probe durch das Rohr ein.

In der folgenden Tabelle wird eine Übersicht zu Beispielen für die Probeneingabe anhand von ausgewählten Blutgasanalysatoren gegeben. In der Tabelle bedeutet "Ja", dass die jeweils angeführte Weise der Probeeingabe mit dem betreffenden Analysator möglich ist.

**Tabelle: Beispiele für die Probeneingabe anhand ausgewählter Blutgasanalysatoren**

| Analysator | Einfüllmund | | | Einsaugrohr für Spritze |
|---|---|---|---|---|
| | Einsaugen aus Kapillare | Einsaugen aus Spritze | Einspritzen mit Spritze | |
| cobas® b 121 / OMNI C | Ja | Nein* | Nein | Ja |
| cobas® b 221 / OMNI S | Ja | Nein* | Ja | Ja |
| cobas® b 123 | Ja | Nein* | Nein | Ja |
| OPTI CCA | Ja | Nein* | Nein | Nein |

| | | | | |
|---|---|---|---|---|
| *Anmerkung: Ja mit zusätzlichem Adapter - für OPTI CCA gibt es einen solchen Adapter | | | | |

Um das Eindringen von Blutgerinnseln und Gewebepartikeln in den Analysator zu verhindern, sind aus dem Stand der Technik sogenannte Koagulatfallen bekannt, die in der englischen Fachliteratur als Clot Catcher bezeichnet werden. Clot Catcher verhindern durch eingebaute Rückhalteelemente, beispielsweise durch ein eingebautes mechanisches Gitter, dass es zu Verstopfungen im Gerät oder zu Messwertverfälschungen durch Blutgerinnsel kommt. Alternativ können auch entsprechende Filter- oder Siebstrukturen sowie weitere dem Fachmann bekannte Rückhalteelemente als Rückhaltevorrichtungen eingesetzt werden.

Derartige Clot Catcher können beispielsweise bei Analysatoren mit Einfüllmund und Betrieb im Einsaugmodus unter Verwendung von Kapillaren als Probengefäßen eingesetzt werden, wobei diese zwischen Kapillare und Einfüllmund angeordnet werden.

In anderen Ausführungsformen können Clot Catcher beispielsweise bei Analysatoren mit Einfüllmund und Betrieb im Einspritzmodus unter Verwendung von Spritzen als Probengefäße eingesetzt werden, wobei diese zwischen Spritze und Einfüllmund angeordnet werden.

Bei Analysatoren mit Probeeingabevorrichtungen über eine Einsaugnadel werden Clot Catcher üblicherweise nicht eingesetzt.

So funktioniert der von Roche Diagnostics vertriebene Clot Catcher uneingeschränkt für Messungen mit Kapillaren und Roche Microsamplern®. In Kombination mit Spritzen funktioniert der Clot Catcher jedoch nur mit Geräten und Betriebsmodi, bei denen ein aktives Einspritzen der Probe des Anwenders möglich ist. Dieser Clot Catcher besteht aus einem thermoplastischen Elastomer (TPE).

Zur Anwendung dieser bekannten Clot Catcher steckt man diesen fest auf das mit Blut gefüllte Probegefäß (beispielsweise eine Kapillare, ein Roche Microsampler® oder eine Spritze). Anschließend wird die Analyse gemäß der jeweiligen Gerätebeschreibung durchgeführt.

Nachteilig an allen aus dem Stand der Technik bekannten Clot Catchern ist, dass diese für Analysatoren, welche durch ihr Design oder die jeweilige Betriebsart bedingt nur ein Einsaugen der Probe ermöglichen, die Verwendung von Spritzen als Probengefäße nicht erlauben.

Aufgabe der vorliegenden Erfindung ist es daher insbesondere, eine Probeneingabevorrichtung bereitzustellen, welche es ermöglicht, Spritzen als Probengefäße in Kombination mit Clot Catchern auch bei Analysatoren zu verwenden, bei denen kein aktives Einspritzen, sondern nur ein Einsaugen der Probe durch das Gerät möglich ist oder welche in dieser Einsaug-Betriebsart betrieben werden.

Die Lösung dieser Aufgabe erfolgt durch Bereitstellen einer Probeneingabevorrichtung mit den kennzeichnenden Merkmalen des Anspruchs 1 zur Eingabe von flüssigen Proben, insbesondere Blutproben, aus Probengefäßen, insbesondere Spritzen, in Analysatoren zur Untersuchung der Zusammensetzung dieser Proben. Diese Probeneingabevorrichtung weist zumindest ein Rückhalteelement auf, welches partikuläre Bestandteile der Probe zumindest teilweise vom Übertritt aus dem Probengefäß in den Analysator zurückhält, wobei diese Probeneingabevorrichtung weiterhin zumindest eine Belüftungsvorrichtung enthält, welche das Entlüften des Probengefäßes während der Eingabe der Probe aus dem Probengefäß in den Analysator ermöglicht, insbesondere bei Einsaugen der Probe durch den Analysator.

In einer möglichen erfindungsgemäßen Ausführungsform wird hierzu als Analysator-Verbindungsteil ein aus dem Stand der Technik bekannter Clot Catcher, beispielsweise ein Clot Catcher von Roche Diagnostics, mit einer Belüftungsvorrichtung, welche eine Belüftung des Probengefäßes bewirkt, zu einer funktionellen Einheit einer Probeneingabevorrichtung kombiniert.

Solche Belüftungsvorrichtungen sind beispielsweise als Belüftungsadapter bzw. als Ansteckteile für Spritzen bekannt, die einen Luftaustausch ermöglichen, während das Analysegerät die Probe einsaugt. Derartige Belüftungsadapter werden beispielsweise von OPTIMedical zusammen mit den Sensorkassetten für das OPTI CCA System vertrieben. Der Belüftungsadapter wird dabei auf die mit Probenmaterial befüllte Spritze aufgesteckt.

Anschließend wird das vordere Teil des Belüftungsadapters an den Einfüllmund des Analysator-Gerätes angesteckt und die Probe durch das Gerät eingesaugt. Während des Einsaugens der Probe in den Analysator wird innerhalb der Spritze die ausgesaugte Probe durch Luft, welche mittels Belüftungsadapter in die Spritze gelangt, ersetzt.

Zweckmäßig ist bei einer erfindungsgemäßen Probeneingabevorrichtung das zumindest eine Rückhaltelement als mechanisches Rückhaltelement, insbesondere als Gitter, Filter oder Siebe ausgebildet.

Ebenso können im Rahmen der Erfindung mehrere Rückhalteelemente in ein und demselben Abschnitt oder auch in räumlich voneinander beabstandeten Abschnitten der Probeneingabevorrichtung vorgesehen sein.

Besonders vorteilhaft ist bei einer erfindungsgemäßen Probeneingabevorrichtung die zumindest eine Belüftungsvorrichtung als Belüftungskanal ausgebildet.

Von der Erfindung sind ebenso Probeneingabevorrichtungen, welche mehrere Belüftungsvorrichtungen aufweisen, mit umfasst. Weiters sind dem Fachmann alternative Belüftungs- bzw. Entlüftungs-Vorrichtungen bekannt und können entsprechend in der erfindungsgemäßen Ausführungsform analog eingesetzt werden. Beispiele für weitere mögliche Belüftungsvorrichtungen sind gasdurchlässige, aber flüssigkeitsundurchlässige Membranen oder Filter oder entsprechende Entlüftungsventile, welche das Eindringen von Luft ermöglichen.

In einer möglichen Ausführungsform werden ein herkömmlicher Clot Catcher als Rückhalteelement und ein Belüftungsadapter, der zwischen Spritze und Clot Catcher angeordnet ist und eine Belüftung der Spritze während des Einsaugens ermöglicht, miteinander verbunden. Diese beiden Komponenten werden fest oder lose zusammengefügt und bilden somit eine entsprechende erfindungsgemäße funktionelle Einheit einer Probeneingabevorrichtung.

Besonders zweckmäßig umfasst eine erfindungsgemäße Probeneingabevorrichtung ein in das Probengefäß, beispielsweise eine Spritze, einführbares Einsaugrohr.

Gemäß der Erfindung sind bei einer Probeneingabevorrichtung die Rückhalteelemente und die Belüftungsvorrichtungen in getrennten Bauteilen verwirklicht, welche in dichtender Weise zu einer funktionellen Einheit assembliert sind, insbesondere durch Verbinden der getrennten Bauteile durch eine Luer-Konus-Verbindung.

Es wird dazu der vordere Teil des Belüftungsadapters den Luer Normen entsprechend angepasst, um das Aufstecken des bestehenden Clot Catcher direkt an den Belüftungsadapter zu ermöglichen. Somit wird eine lösbare Verbindung zwischen den getrennten Bauteilen, dem Analysator-Verbindungsteil (Clot Catcher) und dem Probengefäß-Verbindungsteil (Belühungsadapter), erzielt. Die Fixierung des Analysator-Verbindungsteils, also desjenigen Bauteils, welcher beispielsweise Rückhalteelemente umfasst, am Probengefäß-Verbindungsteil, erfolgt hier nur durch Aufstecken auf dem Luer-Konus des Belüftungsadapters.

Alternativ dazu sind auch Ausführungsformen möglich, bei denen die Fixierung der beiden getrennten Bauteile - des Analysator-Verbindungsteils (Clot Catcher) und des Probengefäß-Verbindungsteils (Belüftungsadapter) - beispielsweise durch zwei Schnapphaken gewährleistet wird. Somit werden die beiden separaten Bauteile unlösbar miteinander verbunden.

Bei der erfindungsgemäßen Probeneingabevorrichtung umfassen die getrennten Bauteile ein Analysator-Verbindungsteil, das mit einem Probeneingang des Analysators verbindbar ist, wobei die Rückhalteelemente im Analysator-Verbindungsteil angeordnet sind.

Vorteilhaft ist bei einer Probeneingabevorrichtung, welche getrennte Bauteile umfasst, ein Probengefäß-Verbindungsteil vorgesehen, welches mit einem Probenauslass des Probengefäßes verbindbar ist, wobei die Belüftungsvorrichtungen im Probengefäß-Verbindungsteil angeordnet sind.

In einer vorteilhaften Ausführungsform der Probeneingabevorrichtung ist zur besseren Haptik und für die Verwendbarkeit mit Luer-Lock-Spritzen ein zusätzlicher Umfangsring beispielsweise am Probengefäß-Verbindungsteil vorgesehen.

Das Analysator-Verbindungsteil ist bevorzugt aus einem Styrol-Ethylen-Butylen-Styrol Material hergestellt.

Als besonders geeignete Materialien zur Fertigung des Probengefäß-Verbindungsteils haben sich beispielsweise Methyl-Methacrylat-Acrylnitril-Butadien-Styrol (MABS), Copolyester oder Polyethylenterephthalat erwiesen. Aufgrund seiner Steifigkeit ist auch Styrol-Acrylnitril (SAN) besonders zur Fertigung des Probengefäß-Verbindungsteils geeignet.

In einer Weiterbildung der Erfindung weist eine Probeneingabevorrichtung eine Probengefäß-Anschlusseinrichtung, insbesondere eine Luer-Konus-Verbindung auf, die mit dem Probengefäß eine lösbare Verbindung bildet.

Besonders zweckmäßig sind die Belüftungsvorrichtungen als Belüftungsschlitze bzw. Belüftungskanäle in der Probengefäß-Anschlusseinrichtung ausgebildet.

In einer Weiterbildung der Erfindung wird ein Verfahren zur Eingabe von flüssigen Proben, insbesondere Blutproben, aus einem Probengefäß, insbesondere einer Spritze, in einen Analysator zur Untersuchung der Zusammensetzung dieser Proben, angegeben, wobei die folgenden Verfahrensschritte durchzuführen sind:
- das Bereitstellen einer erfindungsgemäßen Probeneingabevorrichtung,
- das Verbinden der Probeneingabevorrichtung mit einem Probenauslass des Probengefäßes,
- das Verbinden der Probeneingabevorrichtung mit einem Probeneingang des Analysators,
- das Ansaugen der flüssigen Proben aus dem Probengefäß in den Analysator mittels im Analysator erzeugten Unterdrucks.

Weitere Einzelheiten, Merkmale und Vorteile der Erfindung ergeben sich aus der nachfolgenden Erläuterung der in den Zeichnungen schematisch dargestellten Ausführungsbeispiele. In den Zeichnungen zeigen:
- Fig. 1 eine erste erfindungsgemäße Ausführungsform einer mehrteiligen Probeneingabevorrichtung, mit daran befestigtem Probengefäß sowie an einen Probeneingang eines Analysators angesetzt, jeweils im Längsschnitt in einer Schrägansicht;
- Fig. 2 im Detail ein Probengefäß-Verbindungsteil in einer Schrägansicht von der Seite;
- Fig. 3 das aus Fig. 2 ersichtliche Probengefäß-Verbindungsteil mit daran befestigtem Analysator-Verbindungsteil in einer Schrägansicht von vorne;
- Fig. 4 in einer stark vereinfachten Explosionsdarstellung eine erfindungsgemäße Probeneingabevorrichtung mit einem Einfüllmund eines Analysators;
- Fig. 5 im Detail eine weitere Ausführungsform eines Probengefäß-Verbindungsteils in einer Schrägansicht von der Seite;
- Fig. 6 eine weitere erfindungsgemäße Ausführungsform einer einteiligen Probeneingabevorrichtung in einer Schrägansicht.

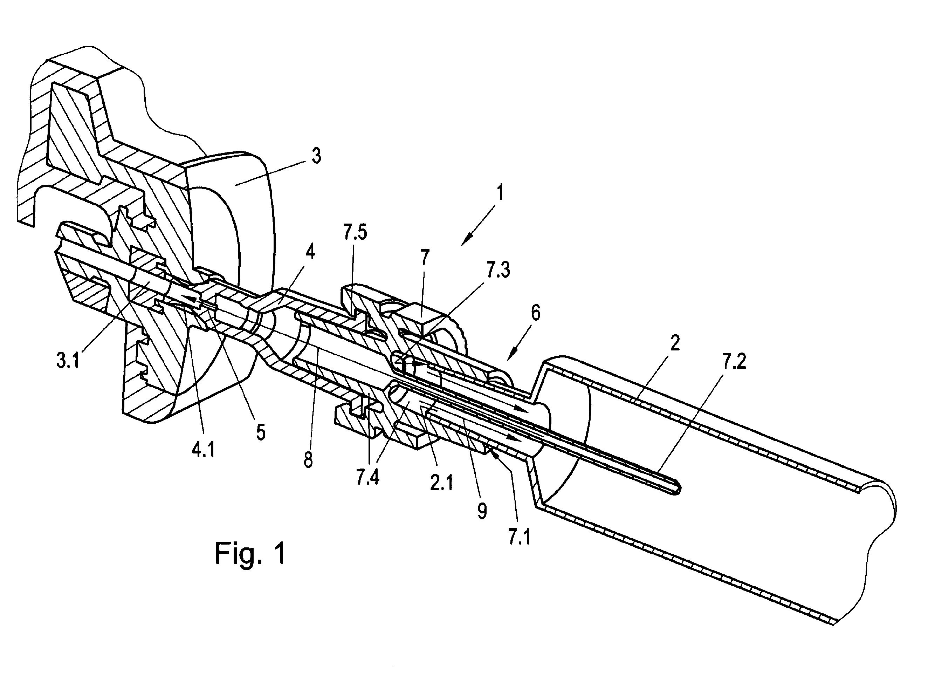

In Fig. 1 ist eine erste erfindungsgemäße Ausführungsform einer mehrteiligen Probeneingabevorrichtung 1 dargestellt. Die Probeneingabevorrichtung 1 ist mit Anschlüssen zur Verbindung zwischen einem Probengefäß 2, beispielsweise einer mit Probematerial befüllte Spritze, und einem Analysator 3 versehen. Die hier gezeigte Probeneingabevorrichtung 1 ist mehrteilig ausgeführt und umfasst ein Analysator-Verbindungsteil 4, welches in seinem Inneren ein Rückhalteelement 5 aufweist. Das Analysator-Verbindungsteil 4 ist weiters mit einem Andockbereich 4.1 an den Analysator 3 versehen.

Das Rückhalteelement 5 ist hier beispielsweise ein mechanisches Gitter, welches partikuläre Bestandteile der Probe zumindest teilweise vom Übertritt aus dem Probengefäß 2 in den Analysator 3 zurückhält.

Handelt es sich bei der zu analysierenden flüssigen Probe im Probengefäß 2 beispielsweise um eine Blutprobe, so werden durch das Rückhalteelement 5 Blutgerinnsel zurückgehalten und es wird somit verhindert, dass es im Analysator-Gerät 3 zu unerwünschten Verstopfungen oder Messwertverfälschungen kommt.

Weiters ist die gezeigte Probeneingabevorrichtung 1 mit Belüftungsvorrichtungen 6 ausgestattet. Dazu sind im Probengefäß-Verbindungsteil 7, an welches ein Probengefäß 2 beispielsweise durch Aufstecken anschließbar ist, beispielsweise mehrere Belüftungskanäle 7.1 zum Belüften des Probengefäßes 2 während der Eingabe der Probe in den Analysator 3 vorgesehen. Während das Probenmaterial aus dem Inneren des Probengefäßes 2 in Pfeilrichtung 8 durch die Probeneingabevorrichtung 1 hindurch zum Probeneingang 3.1 des Analysators 3 gefördert wird, gelangt durch die Belüftungsvorrichtungen 6, also die hier vorgesehenen Belüftungskanäle 7.1, Luft in Pfeilrichtung 9 in das Innere des Probengefäßes 2.

Das Probengefäß-Verbindungsteil 7 weist ein Einsaugrohr 7.2 auf, welches in das Innere des verbundenen Probengefäßes 2, beispielsweise der Spritze, ragt. Weiters ist das Probengefäß-Verbindungsteil 7 mit einer Probengefäß-Anschlusseinrichtung 7.3 versehen, welche hier als ein Luer-Konus 7.4 ausgeführt ist und mit dem das Probengefäß 2, welches im Bereich seiner Probenauslass-Öffnung 2.1 ebenfalls mit einem komplementären Luer-Konus 7.4 versehen ist, eine lösbare Luer-Konus-Verbindung bildet.

Die separaten Bauteile der Probeneingabevorrichtung 1, das Analysator-Verbindungsteil 4 sowie das Probengefäß-Verbindungsteil 7, sind mit einer Schnapphaken-Verbindung 7.5 unlösbar miteinander verbunden.

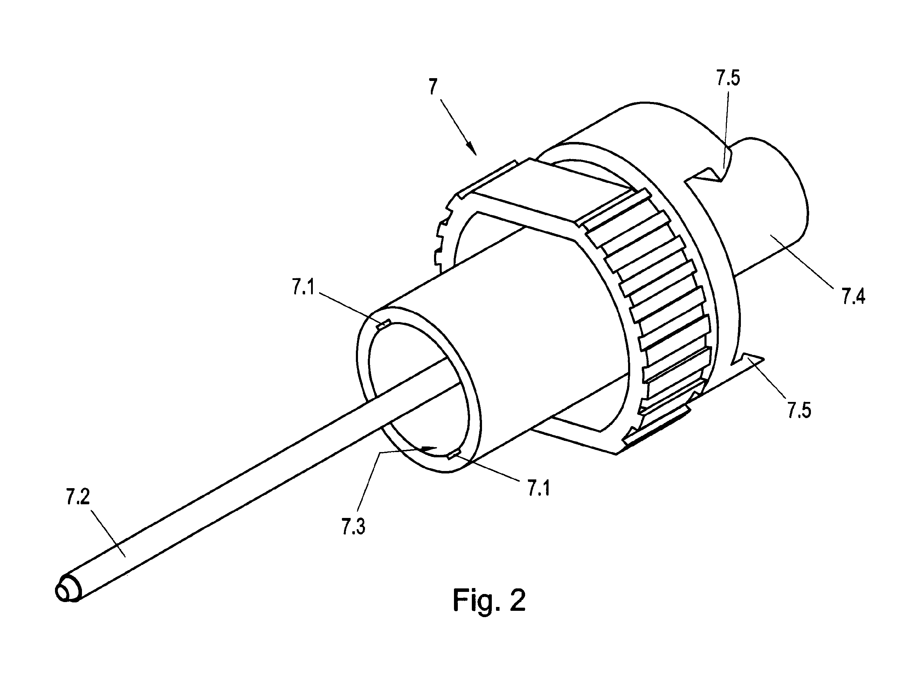

Fig. 2 zeigt im Detail ein Probengefäß-Verbindungsteil 7 in einer Schrägansicht von der Seite. Im Vordergrund sind an der Seite, an der ein nicht dargestelltes Probengefäß, beispielsweise eine Spritze, angesteckt oder aufgeschoben wird, zwei Belüftungskanäle 7.1, die hier als Belüftungsschlitze im Bereich der Probengefäß-Anschlusseinrichtung 7.3 ausgeführt sind, vorgesehen. Ein hohles Einsaugrohr 7.2 dient zum Probentransport und ragt in die flüssige Probe des Probengefäßes. An der in Fig. 2 abgewandten Seite des Probengefäß-Verbindungsteils ist, gegenüberliegend vom Einsaugrohr 7.2, ein Luer-Konus 7.4 zum lösbaren Befestigen eines in Figur 2 nicht dargestellten Analysator-Verbindungsteils, beispielsweise eines Clot Catchers, vorgesehen. Schnapphaken 7.5 dienen zur unlösbaren Verbindung von Probengefäß-Verbindungsteil 7 und Analysator-Verbindungsteil.

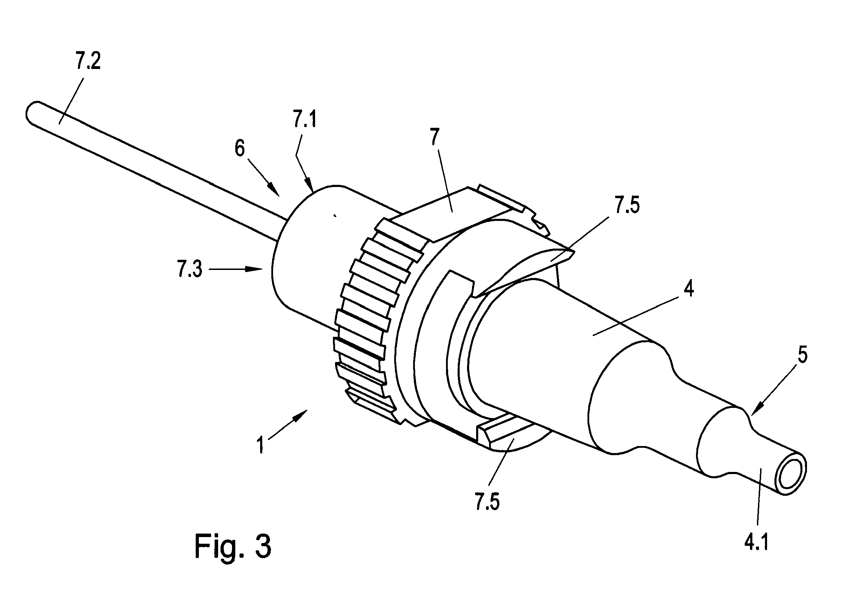

Fig. 3 zeigt das aus Fig. 2 ersichtliche Probengefäß-Verbindungsteil 7 mit daran befestigtem Analysator-Verbindungsteil 4 in einer Schrägansicht von vorne. Das Analysator-Verbindungsteil 4 ist weiters mit einem Andockbereich 4.1 an einen Analysator 3 (hier nicht dargestellt) versehen. Das Rückhalteelement 5 (hier nicht explizit dargestellt und stattdessen dessen räumliche Lage innerhalb des Analysator-Verbindungsteils 4 durch den Pfeil angedeutet) ist hier beispielsweise ein mechanisches Gitter, welches partikuläre Bestandteile der Probe zumindest teilweise vom Übertritt aus dem Probengefäß in den Analysator zurückhält. Neben der Verbindung von Probengefäß-Verbindungsteil 7 mit daran befestigtem Analysator-Verbindungsteil 4 durch eine Luerverbindung erfolgt hier eine zusätzliche Fixierung durch die Schnappverschlüsse 7.5.

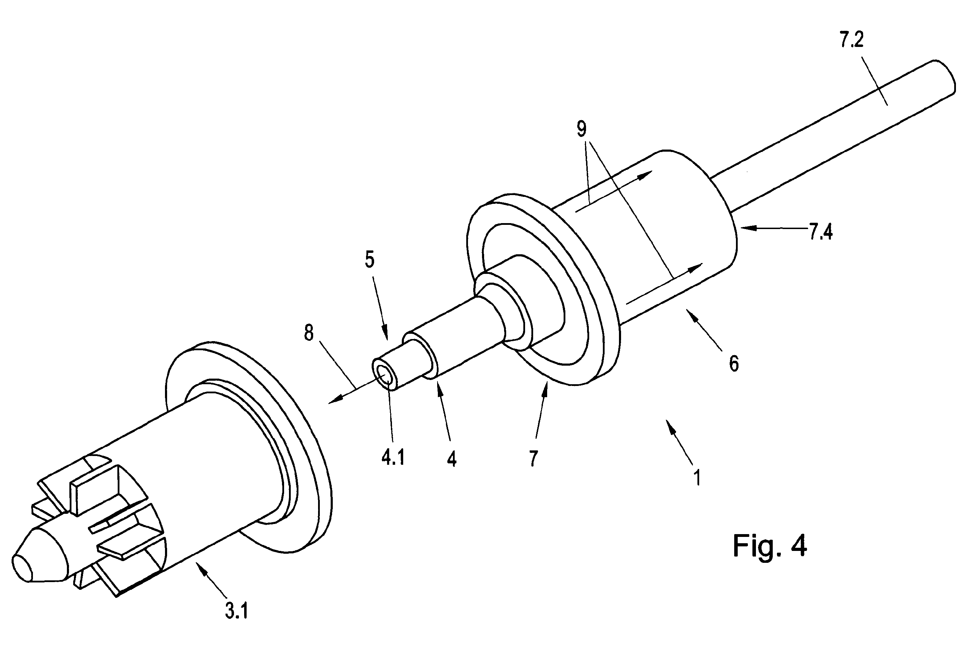

In Fig. 4 ist in einer stark vereinfachten Explosionsdarstellung eine erfindungsgemäße Probeneingabevorrichtung 1 mit einem sogenannten Einfüllmund 3.1, welcher den Probeneingang eines nicht gezeigten Analysators bildet, dargestellt.

Analysator-Verbindungsteil 4 sowie Probe engefäß-Verbindungsteil 7 der Probeneingabevorrichtung 1 sind hier miteinander verbunden dargestellt. Das Analysator-Verbindungsteil 4 umfasst mehrere Rückhalteelemente 5, das Probengefäß-Verbindungsteil 7 ist mit Belüftungsvorrichtungen 6 ausgestattet. Das im Bildvordergrund gezeigte freie Ende des Analysator-Verbindungsteils 4 weist einen Andockbereich 4.1 für einen Analysator auf. Das im Bildhintergrund gelegene, gegenüberliegende freie Ende der Probeneingabevorrichtung 1, an welches ein Probegefäß anschließbar ist, ist mit einem Einsaugrohr 7.2 sowie mit einem Luer-Konus 7.4 zum lösbaren Verbinden mit dem Probegefäß versehen.

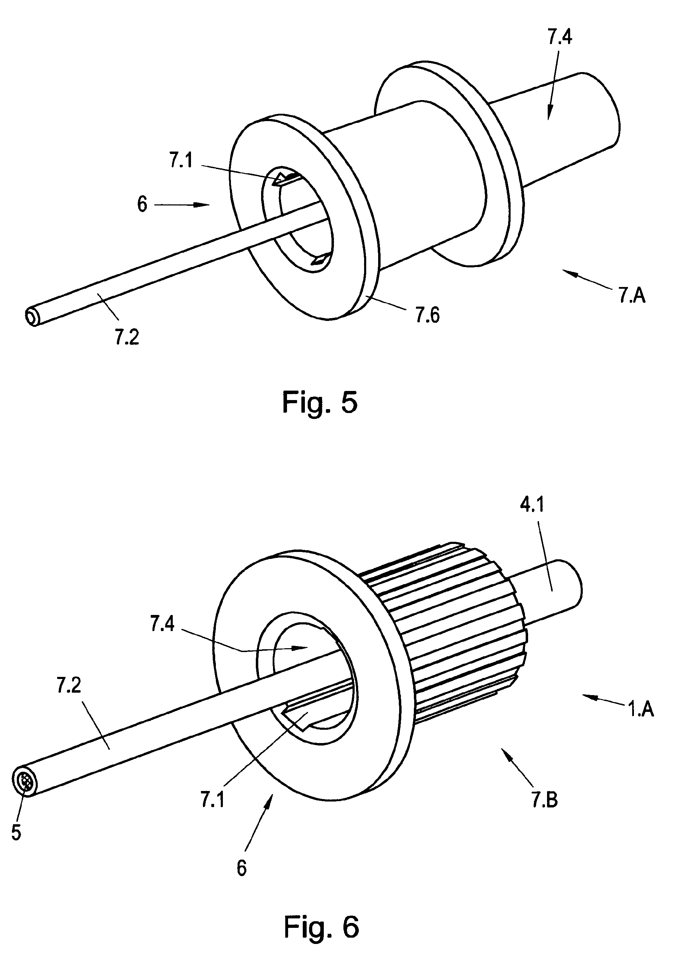

In Fig. 5 ist im Detail eine weitere Ausführungsform eines Probengefäß-Verbindungsteils 7.A in einer Schrägansicht von der Seite dargestellt. Es ist hier ein zusätzlicher Umfangsring 7.6 für eine verbesserte Haptik sowie die Verwendbarkeit mit Luer-Lock Spritzen vorgesehen.

Fig. 6 stellt eine nicht erfindungsgemäße Ausführungsform einer einteiligen Probeneingabevorrichtung 1.A in einer Schrägansicht dar. Hier ist das Probengefäß-Verbindungsteil 7.B an seinem freien Ende mit einem Einsaugrohr 7.2 ausgestattet, welches in seinem Inneren mit einem Rückhalteelement 5 versehen ist. Somit wird neben der Belüftungsfunktion auch die Funktion eines Clot Catchers vom Probengefäß-Verbindungsteil 7.B übernommen. Das dem Einsaugrohr 7.2 gegenüberliegende freie Ende der einteiligen Probeneingabevorrichtung 1.A kann daher direkt an seinem Analysator-Verbindungsteil 4.1 mit einem nicht dargestellten Analysator verbunden werden. Ein separates Analysator-Verbindungsteil, beispielsweise ein bekannter Clot Catcher, muss hier nicht verwendet werden.

Selbstverständlich ist es im Rahmen der Erfindung auch möglich, die Probeneingabevorrichtung 1.A auch mit einem Analysator-Verbindungsteil zu kombinieren und solcherart mehrere, räumlich voneinander beabstandete Rückhalteelemente bei einer Probeneingabevorrichtung zu realisieren.

### Liste der Positionsnummern:

- 1: Probeneingabevorrichtung; bzw. Variante 1.A
- 2: Probengefäß (Spritze)
- 2.1: Probenauslass
- 3: Analysator
- 3.1: Probeneingang
- 4: Analysator-Verbindungsteil (Clot Catcher)
- 4.1: Andockbereich an Analysator
- 5: Rückhalteelement
- 6: Belüftungsvorrichtung
- 7: Probengefäß-Verbindungsteil (Belüftungsadapter); bzw. Varianten 7.A; 7.B
- 7.1: Belüftungskanal
- 7.2: Einsaugrohr
- 7.3: Probengefäß-Anschlusseinrichtung
- 7.4: Luer-Konus-Verbindung
- 7.5: Schnapphaken
- 7.6: Umfangsring
- 8: Probenweg (Pfeilrichtung)
- 9: Luftweg (Pfeilrichtung)

## Patentansprüche

1. Probeneingabevorrichtung (1; 1.A) zur Eingabe von flüssigen Proben, insbesondere Blutproben, aus Probengefäßen (2), insbesondere Spritzen, in Analysatoren (3) zur Untersuchung der Zusammensetzung dieser Proben,
wobei diese Probeneingabevorrichtung (1; 1.A) zumindest ein Rückhalteelement (5) aufweist, welches partikuläre Bestandteile der Probe zumindest teilweise vom Übertritt aus dem Probengefäß (2) in den Analysator (3) zurückhält und
wobei diese Probeneingabevorrichtung (1; 1.A) weiters zumindest eine Belüftungsvorrichtung (6) enthält, welche das Belüften des Probengefäßes (2) während der Eingabe der Probe aus dem Probengefäß (2) in den Analysator (3) ermöglicht, insbesondere bei Einsaugen der Probe durch den Analysator (3),
**dadurch gekennzeichnet, dass** die Rückhaltelemente (5) und die Belüftungsvorrichtungen (6) in getrennten Bauteilen (4; 7) verwirklicht sind, welche in dichtender Weise zu einer funktionellen Einheit assembliert sind, insbesondere durch Verbinden der getrennten Bauteile (4; 7) durch eine Luer-Konus-Verbindung (7.4), und dass die Rückhalteelemente (5) in einem Analysator-Verbindungsteil (4) angeordnet sind, das mit einem Probeneingang (3.1) des Analysators (3) verbindbar ist.

2. Probeneingabevorrichtung (1; 1.A) gemäß Anspruch 1, **dadurch gekennzeichnet, dass** das zumindest eine Rückhaltelement (5) als mechanisches Rückhaltelement, insbesondere als Gitter, Filter oder Sieb ausgebildet ist.

3. Probeneingabevorrichtung (1; 1.A) gemäß Anspruch 1 oder 2, **dadurch gekennzeichnet, dass** die zumindest eine Belüftungsvorrichtung (6) als Belüftungskanal (7.1) ausgebildet ist.

4. Probeneingabevorrichtung (1; 1.A) gemäß einem der vorhergehenden Ansprüche, **gekennzeichnet durch** ein in das Probengefäß (2) einführbares Einsaugrohr (7.2).

5. Probeneingabevorrichtung (1) gemäß einem der vorhergehenden Ansprüche, **dadurch gekennzeichnet, dass** die getrennten Bauteile (4; 7) unlösbar miteinander verbunden sind, insbesondere durch Schnapphaken-Verbindungen (7.5).

6. Probeneingabevorrichtung (1) gemäß einem der vorhergehenden Ansprüche, **dadurch gekennzeichnet, dass** die getrennten Bauteile (4; 7) ein Probengefäß-Verbindungsteil (7) umfassen, das mit einem Probenauslass (2.1) des Probengefäßes (2) verbindbar ist, wobei die Belüftungsvorrichtungen (6) im Probengefäß-Verbindungsteil (7) angeordnet sind.

7. Probeneingabevorrichtung (1; 1.A) gemäß einem der vorhergehenden Ansprüche, **dadurch gekennzeichnet, dass** das Probengefäß-Verbindungsteil (7) aus einem Material hergestellt ist, das aus Methyl-Methacrylat-Acrylnitril-Butadien-Styrol (MABS), Copolyester, Polyethylenterephthalat, vorzugsweise jedoch Styrol-Acrylnitril (SAN), ausgewählt ist.

8. Probeneingabevorrichtung (1; 1.A) gemäß einem der vorhergehenden Ansprüche, **dadurch gekennzeichnet, dass** das Analysator-Verbindungsteil (4) aus einem Styrol-Ethylen-Butylen-Styrol Material hergestellt ist.

9. Probeneingabevorrichtung (1; 1.A) gemäß einem der vorhergehenden Ansprüche, **gekennzeichnet durch** eine Probengefäß-Anschlusseinrichtung (7.3), insbesondere eine Luer-Konus-Verbindung (7.4), die mit dem Probengefäß (2) eine lösbare Verbindung bildet.

10. Probeneingabevorrichtung (1; 1.A) gemäß Anspruch 9, **dadurch gekennzeichnet, dass** die Belüftungsvorrichtungen (6) als Belüftungsschlitze (7.1) in der Probengefäß-Anschlusseinrichtung (7.3) ausgebildet sind.

11. Probeneingabevorrichtung (1; 1.A) gemäß einem der vorhergehenden Ansprüche, **gekennzeichnet durch** zumindest einen Umfangsring (7.6).

12. Verfahren zur Eingabe von flüssigen Proben, insbesondere Blutproben, aus einem Probengefäß (2), insbesondere einer Spritze, in einen Analysator (3) zur Untersuchung der Zusammensetzung dieser Proben,
**gekennzeichnet durch**
das Bereitstellen einer Probeneingabevorrichtung (1; 1.A) gemäß einem der vorhergehenden Ansprüche,
das Verbinden der Probeneingabevorrichtung (1; 1.A) mit einem Probenauslass (2.1) des Probengefäßes (2),
das Verbinden der Probeneingabevorrichtung (1; 1.A) mit einem Probeneingang (3.1) des Analysators (3), und
das Ansaugen der flüssigen Proben aus dem Probengefäß (2) in den Analysator (3) mittels im Analysator erzeugten Unterdrucks.

## Claims

1. A sample input device (1; 1.A) for inputting liquid samples, in particular blood samples, from sample containers (2), in particular syringes, into analyser devices (3) for the examination of the composition of these samples,
wherein this sample input device (1; 1.A) has at least one retaining element (5), which retains particulate ingredients of the sample at least in part from passing over from the sample container (2) into the analyser device and
wherein this sample input device (1; 1.A) further contains at least one ventilation device (6), which enables ventilation of the sample container (2) during the input of the sample from the sample container (2) into the analyser device (3), in particular when the analyser device (3) sucks in the sample,
**characterized in that** the retaining elements (5) and the ventilation devices (6) are embodied in separate components (4; 7), which are assembled in a sealed way into a functional unit, in particular by connecting the separate components (4; 7) by way of a Luer cone connection (7.4), and that the retaining elements (5) are arranged in an analyser device connection member (4), which may be connected with a sample inlet (3.1) of the analyser device (3).

2. A sample input device (1; 1.A) according to claim 1, **characterized in that** the at least one retaining element (5) is embodied as a mechanical retaining element, in particular as a lattice, filter or sieve.

3. A sample input device (1; 1.A) according to claim 1 or 2, **characterized in that** the at least one ventilation device (6) is embodied as a ventilation channel (7.1).

4. A sample input device (1; 1.A) according to any of the preceding claims, **characterized by** a suction tube (7.2) that may be introduced in the sample container (2).

5. A sample input device (1) according to any of the preceding claims, **characterized in that** the separate components (4; 7) are connected inseparably with each other, in particular by snap-fit connections (7.5).

6. A sample input device (1; 1.A) according to any of the preceding claims, **characterized in that** the separate components (4; 7) comprise a sample container connection member (7), which may be connected with a sample outlet (2.1) of the sample container (2), wherein the ventilation devices (6) are arranged in the sample container connection member (7).

7. A sample input device (1; 1.A) according to any of the preceding claims, **characterized in that** the sample container connection member (7) is made of a material, which is selected from methyl methacrylate acrylnitrile butadiene styrene (MABS), co-polyester, polyethylene therephthalate, preferably, however, styrene acryl nitrile (SAN).

8. A sample input device (1; 1.A) according to any of the preceding claims, **characterized in that** the analyser device connection member (4) is made of a styrene ethylene butylene styrene material.

9. A sample input device (1; 1.A) according to any of the preceding claims, **characterized by** a sample container connection device (7.3), in particular a Luer cone connection (7.4), which forms a separable connection with the sample container (2).

10. A sample input device (1; 1.A) according to claim 9, **characterized in that** the ventilation devices (6) are embodied as ventilation slits (7.1) in the sample container connection device (7.3).

11. A sample input device (1; 1.A) according to any of the preceding claims, **characterized by** at least one perimeter ring (7.6).

12. A method for inputting liquid samples, in particular blood samples, from a sample container (2), in particular a syringe, into an analyser device (3) for the examination of the composition of these samples,
**characterized by**
making provision of a sample input device (1; 1.A) according to any of the preceding claims, connecting the sample input device (1; 1.A) with a sample outlet (2.1) of the sample container (2),
connecting the sample input device (1; 1.A) with a sample inlet (3.1) of the analyser device (3) and
sucking in the liquid samples from the sample container (2) into the analyser device (3) by means of a negative pressure generated in the analyser device.

## Revendications

1. Dispositif d'introduction d'échantillons (1 ; 1A) pour l'introduction d'échantillons liquides, en particulier des échantillons de sang, provenant de récipients à échantillons (2), en particulier des seringues, dans des analyseurs (3) pour analyser la composition de ces échantillons,
dans lequel le dispositif d'introduction d'échantillons (1 ; 1.A) comprend au moins un élément de retenue (5) qui retient les composants particulaires de l'échantillon au moins temporairement à l'encontre d'un passage hors du récipient à échantillons (2) jusque dans l'analyseur (3), et
dans lequel le dispositif d'introduction d'échantillons (1 ; 1.A) contient en outre au moins un dispositif d'aération (6) qui permet l'aération du récipient à échantillons (2) pendant l'introduction de l'échantillon depuis le récipient à échantillons (2) jusque dans l'analyseur (3), en particulier lors de l'aspiration de l'échantillon par l'analyseur (3), **caractérisé en ce que** les éléments de retenue (5) et les dispositifs d'aération (6) sont réalisés dans des composants séparés (4 ; 7) qui sont assemblés d'une manière étanche pour donner une unité fonctionnelle, en particulier par liaison des composants séparés (4 ; 7) par une liaison de type cône Luer (7.4), et **en ce que** les éléments de retenue (5) sont agencés dans une pièce de liaison (4) à l'analyseur, qui est susceptible d'être reliée à une entrée à échantillons (3.1) de l'analyseur (3).

2. Dispositif d'introduction d'échantillons (1 ; 1.A) selon la revendication 1, **caractérisé en ce que** ledit au moins un élément de retenue (5) est réalisé sous forme d'élément de retenue mécanique, en particulier sous forme de grille, de filtre ou de crible.

3. Dispositif d'introduction d'échantillons (1 ; 1.A) selon la revendication 1 ou 2, **caractérisé en ce que** ledit au moins un dispositif d'aération (6) est réalisé sous forme de canal d'aération (7.1).

4. Dispositif d'introduction d'échantillons (1 ; 1.A) selon l'une des revendications précédentes, **caractérisé par** un tube d'aspiration (7.2) susceptible d'être introduit dans le récipient à échantillons (2).

5. Dispositif d'introduction d'échantillons (1) selon l'une des revendications précédentes, **caractérisé en ce que** les composants séparés (4 ; 7) sont reliés les uns aux autres sans pouvoir être détachés, en particulier par des liaisons à crochets agrippants (7.5).

6. Dispositif d'introduction d'échantillons (1) selon l'une des revendications précédentes, **caractérisé en ce que** les composants séparés (4 ; 7) comprennent une pièce de liaison (7) au récipient à échantillons, qui est susceptible d'être reliée à une sortie à échantillons (2.1) du récipient à échantillons (2), et les dispositifs d'aération (6) sont agencés dans la pièce de liaison (7) au récipient à échantillons.

7. Dispositif d'introduction d'échantillons (1:1.A) selon l'une des revendications précédentes, **caractérisé en ce que** la pièce de liaison (7) au récipient à échantillons est fabriquée en un matériau qui est choisi parmi méthyle-méthacrylate-acrylonitrile-butadiène-styrène (MABS), copolyester, polyéthylène téréphtalate, mais de préférence styrène-acrylonitrile (SAN).

8. Dispositif d'introduction d'échantillons (1 ; 1.A) selon l'une des revendications précédentes, **caractérisé en ce que** la pièce de liaison (4) à l'analyseur est fabriquée en un matériau styrène-éthylène-butylène-styrène.

9. Dispositif d'introduction d'échantillons (1 ; 1.A) selon l'une des revendications précédentes,
**caractérisé par** un système de raccordement (7.3) au récipient à échantillons, en particulier une liaison de type cône Luer (7.4) qui forme une liaison détachable avec le récipient à échantillons (2).

10. Dispositif d'introduction d'échantillons (1 ; 1.A) selon la revendication 9, **caractérisé en ce que** les dispositifs d'aération (6) sont réalisés sous forme de fentes d'aération (7.1) dans le système de raccordement (7.3) au récipient à échantillons.

11. Dispositif d'introduction d'échantillons (1 ; 1.A) selon l'une des revendications précédentes, **caractérisé par** au moins une bague périphérique (7.6).

12. Procédé pour l'introduction d'échantillons liquides, en particulier d'échantillons de sang, hors d'un récipient à échantillons (2), en particulier une seringue, jusque dans un analyseur (3) pour analyser la composition de ces échantillons,
**caractérisé par** les étapes consistant à
préparer un dispositif d'introduction d'échantillons (1 ; 1.A) selon l'une des revendications précédentes,
relier le dispositif d'introduction d'échantillons (1 ; 1.A) avec une sortie à échantillon (2.1) du récipient à échantillon (2),
relier le dispositif d'introduction d'échantillons (1 ; 1.A) avec une entrée à échantillons (3.1) de l'analyseur (3), et
aspirer les échantillons liquides hors du récipient à échantillons (2) jusque dans l'analyseur (3) au moyen d'une dépression générée dans l'analyseur.
